# EUROPEAN PATENT SPECIFICATION

(11) **EP 4 539 910 B1**
(45) Date of publication and mention of the grant of the patent: **11.02.2026**
(21) Application number: 23739658.5
(22) Date of filing: 14.06.2023
(51) Int. Cl.: A61M 16/00, A61M 16/12, A61M 16/20

(54) **LUNG VENTILATOR**
LUNGENBEATMUNGSGERÄT
VENTILATEUR PULMONAIRE

(30) Priority: 15.06.2022 IT 202200012644
(43) Date of publication of application: 23.04.2025
(73) Proprietor: Tecnologie Meccaniche S.p.A., 00041 Albano Laziale (RM) (IT)
(72) Inventor: TINTI, Gianfranco, 00041 Albano Laziale (RM) (IT); TINTI, Vittoriano, 00041 Albano Laziale (RM) (IT); TINTI, Emanuele, 00041 Albano Laziale (RM) (IT); TINTI, Massimiliano, 00041 Albano Laziale (RM) (IT)
(74) Representative: IP Sextant s.r.l.
(86) International application number: PCT/IB2023/056123
(87) International publication number: WO 2023/242748

(56) References cited:
- US-A- 4 036 221
- US-A- 5 044 362
- US-A- 5 531 221
- US-A1- 2009 241 953
- US-A1- 2021 338 952

## Description

### BACKGROUND OF THE INVENTION

The present invention relates to a pulmonary ventilator, particularly suitable for being used for patients with respiratory failure and/or controlled ventilation of patients in intensive care unit or surgery room.

### PRIOR ART

In the state-of-the-art ventilators are known which are used to provide breathing support to patients. Such pulmonary ventilators, for example such as those disclosed in US2009/241953 A1, US2021/338952 A1, US4036221 A and US5044362 A, each comprise an electromechanical apparatus configured to receive air from a source external with respect to the apparatus, mix it with oxygen, and send it towards the patient's respiratory system. The operation of such an electromechanical apparatus is assisted by sensors and actuators (for example, valves, heaters, humidifiers, etc.), which are incorporated in the apparatus, configured to monitor the physical parameters of the thus obtained air-oxygen mixture to be supplied to the patient and modify them, if necessary, so that the air-oxygen mixture can reach the patient's lungs in conditions that are compatible with the physiological respiratory act. On the market, traditional pulmonary ventilators can be distinguished between fixed ventilators or portable ventilators which are different in terms of architecture and characteristics. Traditional pulmonary ventilators (both fixed and portable) can also be divided into volume-controlled ventilators and pressure-controlled ventilators.

Volume-controlled ventilators are configured to regulate a volume of a ventilation gas to be delivered to the patient during the respiratory act. That volume corresponds to a tidal volume set by a healthcare professional, i.e. a determined quantity of air-oxygen mixture which enters and leaves the lungs with each respiratory act. These volume-controlled ventilators suffer from some drawbacks. In fact, they do not take into account the features of the respiratory system (airway resistance or pulmonary compliance) of the patient undergoing ventilation, therefore:
- a decrease in pulmonary compliance of the patient undergoing ventilation, due to restrictive processes, can cause rupture of the pulmonary alveoli (barotrauma) due to the disproportionate increase in interalveolar pressure; and
- an increase in the patient's lung compliance, or a ventilation gas leakage from the ventilation duct connected to the patient, for example due to a leak, can lead to poor oxygenation (hypoxia), as the interalveolar pressure does not reach sufficiently adequate levels to guarantee alveolar gas exchange.

Conversely, pressure-controlled ventilators are able to compensate for small leaks that can occur in the ventilation circuit. However, the tidal volume delivered by them varies significantly as the features of the patient's respiratory system vary. In particular:
- an increase in respiratory resistance and/or decrease in compliance, may lead to a risk of hypoventilation, while
- a decrease in respiratory resistance and/or an increase in compliance can lead to volotrauma (excessive tidal volume).

An example of a traditional ventilator is disclosed in US patent no. US5531221. In US5531221 the pulmonary ventilator uses a double delivery cylinder-piston unit, connected between a ventilation gas suction circuit to be delivered to a patient and a gas delivery circuit. This ventilator also comprises an electric motor unit and one shaft, the latter connected in a known manner on one side to the electric motor and on the other rigidly connected to the piston of the cylinder-piston unit. The overall dimension of this ventilator, which also depends on the non-negligible overall dimension of the electric motor driving the cylinder-piston unit, makes it difficult to be used as a portable ventilator. Not only that, the movement of the shaft connected to the piston in and out of the cylinder-piston unit exposes the mixture contained therein to possible contamination.

This type of ventilator performs very accurate control of the pressure of the ventilation gas delivered to the patient, so that it is at a value compatible with physiological respiratory act, and only approximately controls the amount (volume) of ventilation gas actually delivered to the patient.

In other traditional ventilators, measurement of the volume of ventilation gas is relied exclusively on volumetric/mass type sensors, which can suffer from time drift phenomena, making their periodic calibration necessary. Those sensors usually perform an indirect measurement of the volume of the ventilation gas delivered, based on the measurement of the temperature variation of an object hit by the flow of that ventilation gas inspired or exhaled by the patient, and this makes the measurement of volume suffer from a certain "physiological" degree of inaccuracy. Other more advanced ventilators perform measurements of the pressure, flow and volume of the ventilation gas delivered to the patient, using a single sensor in proximity of the patient's airways, exploiting the Venturi effect. Even these ventilators are not exempt from the need for periodic calibration. Last but not least, the aforementioned sensors can have different features and sensitivity depending on the volume to be measured (for example, whether employed in ventilators for pediatric use or not), making the device not very versatile in its use.

### OBJECTS OF THE INVENTION

The need to improve the state of the art in the assisted ventilation sector is therefore felt, and the main purpose of the present invention is to provide a pulmonary ventilator which is able to control easily and in a more precise and reliable way both the pressure and the volume of gas delivered to a patient, compared to traditional ventilators,.

Another object of the present invention is to provide a pulmonary ventilator which has reduced consumption and reduced overall dimensions compared to traditional ventilators.

A further object of the present invention is to provide a pulmonary ventilator which is safer than traditional ventilators in terms of contamination of the gases circulating therein.

The invention is defined by the appended claims.

More particularly, the invention relates to a pulmonary ventilator configured for delivering to a patient, for each respiratory act, a desired volume of a ventilation gas at a desired pressure, said pulmonary ventilator having a ventilation outlet, configured for being put in fluid communication with respiratory tract of said patient, and an exhaust outlet, in communication with the outside of said pulmonary ventilator, said pulmonary ventilator comprising at least one adjustment circuit, having:
- a cylinder-piston unit having a cylinder and a piston which delimit:
   - an actuation chamber, having an inlet and an outlet, wherein said inlet of said actuation chamber is configured for selectively receiving a first gas under pressure, and said outlet of said actuation chamber is in fluid communication with said exhaust outlet; and
   - a ventilation chamber, having an inlet and an outlet, wherein said inlet of said ventilation chamber is configured for selectively receiving said ventilation gas under pressure to be provided to said patient, and said outlet of said ventilation chamber is in fluid communication with said ventilation outlet of said pulmonary ventilator;
- at least one device for adjusting the flow entering into said actuation chamber and at least one a device for adjusting the flow leaving said actuation chamber, at least one device for adjusting the flow entering into said ventilation chamber and at least one device for adjusting the flow leaving said ventilation chamber;
- at least one pressure detecting device, configured for detecting in use the pressure of said first gas in said actuation chamber and another pressure detecting device, configured for detecting in use the pressure of said ventilation gas in said ventilation chamber;
- one position detector, configured for detecting the position of said piston inside said cylinder;
- one acquisition and processing unit, operatively connected to said position detector, each device for adjusting the flow and each pressure detecting device
wherein said acquisition and processing unit is configured for:
- acquiring and processing input signals regarding the position of said piston in said cylinder, the pressure of said first gas in said actuation chamber and the pressure of said ventilation gas in said ventilation chamber; and
providing output signals for controlling the opening-closing of each device for adjusting the flow entering into and leaving said actuation chamber and the opening-closing of each device for adjusting the flow entering into and leaving said ventilation chamber, based on said position of said piston in said cylinder, said pressure of said first gas in said actuation chamber and said pressure of said ventilation gas in said ventilation chamber, so that one quantity of said ventilation gas corresponding to at least said desired volume enters into said ventilation chamber and then said desired volume of said ventilation gas leaves said ventilation chamber toward said ventilation outlet at said desired pressure.

According to another aspect of the invention, said piston can be a magnetized piston, configured for being moved inside said cylinder due to the pressure difference between said first gas in said actuation chamber and said ventilation gas in said ventilation chamber.

According to a further aspect of the invention, said position detector can be externally fixed to said cylinder and can be configured for magnetically detecting the position of said piston in said cylinder.

According to an additional aspect of the invention, said pulmonary ventilator can comprise at least one first inlet, configured for being placed in fluid communication with a source of a first gas under pressure, wherein said source can be a primary source external to said pulmonary ventilator and wherein said inlet of said actuation chamber can be configured for being selectively placed in fluid communication with said first inlet of said pulmonary ventilator for selectively receiving said first gas under pressure.

According to another aspect of the invention, said pulmonary ventilator can have one second inlet and comprise at least one mixing circuit, said at least one mixing circuit including:
- one mixing chamber;
- a first inlet in said mixing chamber, said first inlet of said mixing chamber being in fluid communication with said first inlet of said pulmonary ventilator and configured for receiving said first gas under pressure, when provided by said primary source;
- a second inlet in said mixing chamber, said second inlet of said mixing chamber being in fluid communication with said second inlet of said pulmonary ventilator and configured for receiving a second gas under pressure; and
- a first outlet of said mixing chamber, in fluid communication with said inlet of said ventilation chamber,
said at least one mixing circuit being operatively controlled by said acquisition and processing unit and configured for mixing said first gas under pressure and sad second gas under pressure therebetween, thereby obtaining said ventilation gas.

According to a further aspect of the invention, said pulmonary ventilator can comprise one auxiliary circuit for supplying said first gas under pressure, said auxiliary circuit comprising:
- one pressurized storage group of said first gas under pressure; and
- a first outlet, in fluid communication with said storage group on one side and, on the other side, selectively in fluid communication with said inlet of said actuation chamber, said first outlet of said auxiliary circuit being operatively controlled by said acquisition and processing unit and configured for being closed, when said primary source of said first gas under pressure is connected to said first inlet, or opened, when said primary source of said first gas under pressure is disconnected from said first inlet .

According to an additional aspect of the invention, said mixing circuit can comprise a third inlet in said mixing chamber, and said auxiliary circuit for supplying said first gas under pressure can comprise at least one second outlet, in fluid communication with said storage group on one side and, on the other side, in fluid communication with said third inlet of said mixing circuit, said second outlet being operatively controlled by said acquisition and processing unit and configured for being closed, when said primary source of said first gas under pressure is connected to said first inlet, or opened, when said primary source of said first gas under pressure is disconnected from said first inlet, so that said first gas under pressure in said storage group can be supplied in said mixing chamber .

According to another aspect of the invention, said acquisition and processing unit can comprise at least two programmable electronic systems in communication with each other, which are configured to implement a hardware and control redundancy.

### BRIEF DESCRIPTION OF THE DRAWINGS

The present invention will now be described, by way of illustration and not limitation, according to its preferred embodiments, with particular reference to the drawings in the attached Figures, wherein:
Figure 1 shows a diagram of the main blocks of a pulmonary ventilator according to a preferred embodiment of the present invention;
Figure 2 is a pneumatic diagram of the pressure and volume adjustment circuit for the ventilation gas of the pulmonary ventilator of Figure 1;
Figure 3 illustrates a pneumatic diagram of the ventilation gas mixing circuit of the pulmonary ventilator of Figure 1; and
Figure 4 shows a pneumatic diagram of an auxiliary circuit for delivering a first gas used by the pulmonary ventilator of Figure 1.

### EMBODIMENTS OF THE INVENTION

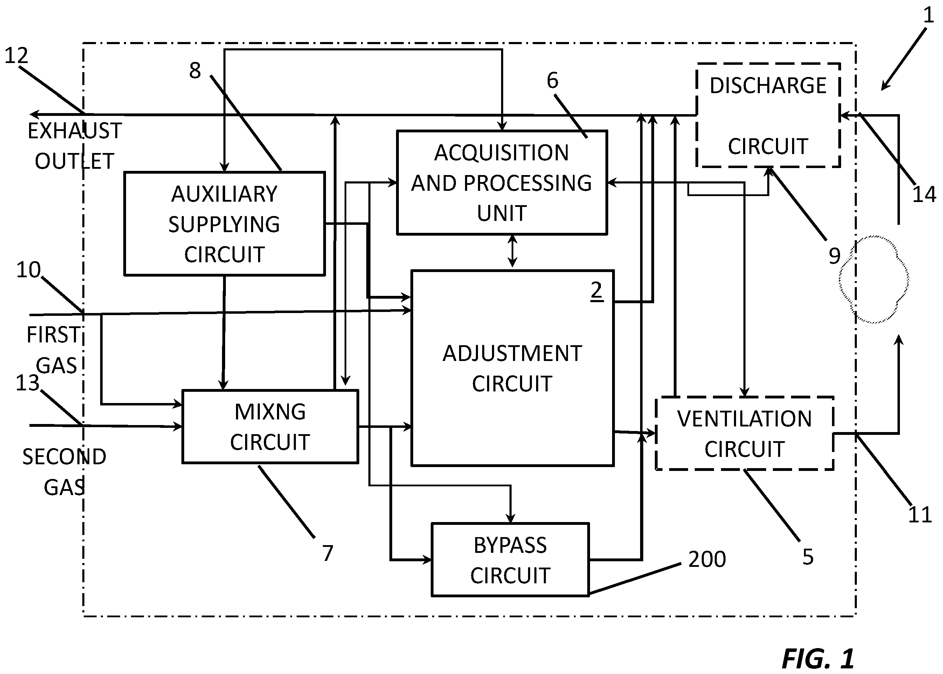

With reference to the accompanying figures, in particular to Figure 1, it will be noted that a pulmonary ventilator according to a preferred embodiment of the present invention is indicated with the reference number 1 and comprises one ventilation outlet 11, configured for being put in fluid communication with the respiratory system of a patient (where Figure 1 schematically represents a ventilation mask ) and to deliver toward said patient one ventilation gas, and one exhaust outlet 12, in fluid communication with the outside of pulmonary ventilator, also configured for being put in fluid communication also with the respiratory system of a patient, for example through a discharge inlet 14 of the pulmonary ventilator 1 and a suitable discharge circuit 9.

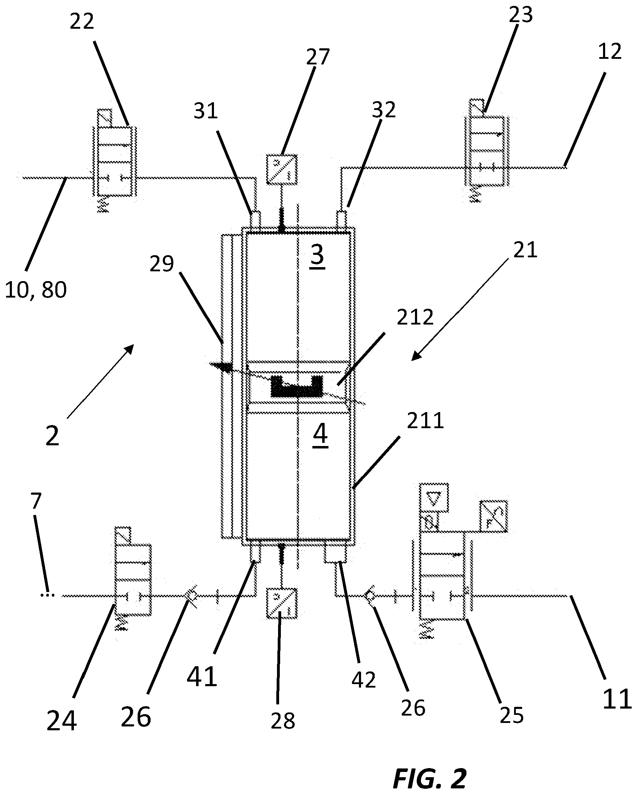

The pulmonary ventilator 1 advantageously comprises at least one adjustment circuit 2, configured for adjusting the volume and the pressure of the ventilation gas to be provided to the patient at each respiratory act. Said adjustment circuit 2 includes (see in particular Figure 2) a cylinder-piston unit 21 having a cylinder 211 and a piston 212 which delimit an actuation chamber 3, having an inlet 31 and an outlet 32, and a ventilation chamber 4, having an inlet 41 and an outlet 42.

The inlet 31 of the actuation chamber 3, as will be explained in the following, is configured for selectively receiving a first gas under pressure, while the outlet 32 of the actuation chamber 3 is in fluid communication with the exhaust outlet 12 of the pulmonary ventilator 1.

The inlet 41 of the ventilation chamber 4 is configured for selectively receiving the ventilation gas under pressure to be delivered to the patient, obtained within the pulmonary ventilator in a way that will be described in the following, and the outlet 42 of the ventilation chamber 4 is in fluid communication with the ventilation outlet 11 of the pulmonary ventilator 1, optionally, as illustrated in Figure 1, through a suitable ventilation circuit.

The adjustment circuit 2 of the pulmonary ventilator 1 also comprises at least one device 22 for adjusting the flow entering the actuation chamber 3 and at least one device 23 for adjusting the flow leaving the actuation chamber 3, as well as at least one device 24 for adjusting the flow entering the ventilation chamber 4 and at least one device 25 for adjusting the flow leaving the ventilation chamber 4.

According to a particularly preferred embodiment of the invention, the device 22 for adjusting the flow entering the actuation chamber 3 and the device 23 for adjusting the flow leaving the actuation chamber 3 comprise proportional two-way directional control valves. Still according to a particularly preferred embodiment of the invention, the device 24 for adjusting the flow entering the ventilation chamber 4 comprises at least one on-off control valve and the device 25 for adjusting the flow leaving the ventilation chamber 4 comprises at least one proportional flow control valve. Between the device 24 for adjusting the flow entering the ventilation chamber 4 and the inlet 41 of said ventilation chamber 4, as well as between the outlet 42 of the ventilation chamber 4 and the device 25 for adjusting the flow leaving said ventilation chamber 4 the adjustment circuit 2 also comprises one-way valves 26, which allow unidirectionality of the ventilation gas towards the ventilation chamber 4 and from that to the outlet 11 of the pulmonary ventilator 1.

According to the present invention, the adjustment circuit 2 of the pulmonary ventilator 1 also comprises at least one pressure detecting device 27 in the actuation chamber 3 and a pressure detecting device 28 in the ventilation chamber 4. The pressure detecting device 27 in the actuation chamber 3 is configured for detecting, both continuously or at steps, for example at prefixed time instants depending on the needs, the pressure value of the first gas, while the pressure detecting device 28 in the ventilation chamber 4 is configured for detecting, both continuously or at steps, for example at prefixed time instants depending on the needs, the pressure value of the ventilation gas.

According to a particularly advantageous aspect, the adjustment circuit 2 of the pulmonary ventilator 1 also comprises a position detector 29, configured for detecting the position of the piston 212 inside the cylinder 211 with respect to a reference, for example with respect to a central cross-section of the cylinder or with respect to an end cross-section thereof (for example coincident with the bottom or the top of the cylinder represented in Figure 2).

The pulmonary ventilator 1 also comprises one acquisition and processing unit 6, for example having at least two programmable electronic systems in communication with each other, which implement hardware and control redundancy, to guarantee safety and operation of the pulmonary ventilator 1 even in the event of failure of one or the other of these programmable electronic systems. Such acquisition and processing unit 6 is operatively connected to position detector 29, each device (22, 23, 24, 25) for adjusting the flow and each pressure detecting device 27 and 28 of the actuation chamber 3 and ventilation chamber 4 of the cylinder-piston unit21.

The acquisition and processing unit 6 is advantageously configured for acquiring and processing suitable input signals regarding the position of the piston 212 in the cylinder 211, the pressure value of the first gas in the actuation chamber 3 and the pressure value of the ventilation gas in the ventilation chamber 4 and to send suitable output signals for controlling the opening-closing of each device (22, 23) for adjusting the flow entering and leaving the actuation chamber 3 and each device (24, 25) for adjusting the flow entering and leaving said ventilation chamber 4, based on the position of the piston 212 in the cylinder 211, the pressure values of the gases in the actuation chamber 3 and in the ventilation chamber 4, so that one quantity of the ventilation gas corresponding to at least the desired volume Vd enters the ventilation chamber 4 and then the desired volume Vd of the ventilation gas leaves the ventilation chamber 4, toward the ventilation outlet 11, at a desired pressure Pd.

In particular, the desired volume Vd corresponds to a tidal volume value, automatically determined by the acquisition and processing unit 6 or manually set by one healthcare operator and stored in the acquisition and processing unit 6 of the pulmonary ventilator 1 in any suitable way, for example through a keyboard or a touch screen or other input/output device that can be connected to the pulmonary ventilator 1 or that is integrated therein. The desired pressure Pd corresponds to a pressure value within a safety range, compatible with the respiratory act of the patient, automatically determined by the acquisition and processing unit 6 or manually set by one healthcare operator and stored in the acquisition and processing unit 6 of the pulmonary ventilator 1 in any suitable way, for example through the abovesaid keyboard or touch screen or other input/output device that can be connected to the pulmonary ventilator 1 or that is integrated therein.

According to a particularly advantageous aspect, the piston 212 of the cylinder-piston unit 2 is a magnetized piston, configured for being moved in the cylinder 211, for example along suitable guides within the cylinder 211, due to the pressure difference between the first gas in the actuation chamber 3 and the pressure value of the ventilation gas in the ventilation chamber 4. Moreover, the position detector 29 is fixed external to the cylinder 211 and is configured for magnetically detecting the position of the piston 212 in the cylinder 211. The configuration of the cylinder-piston unit 2, wherein the piston is not mechanically actuated through a shaft connected thereto as in the prior art and instead it slides on suitable guides due to the pressure difference between the two chambers, is advantageous with respect to the prior art, given that the cylinder is closed (except for the gas inlets and outlets of the actuation chamber and the ventilation chambers) and, accordingly, the possibility of contamination from the outside of the gas circulating therewithin is reduced. Not only that, as will be better explained in the following, the configuration of the cylinder-piston unit 2 allows storing a precise desired volume Vd of ventilation gas (the tidal volume set by the operator) in the ventilation chamber, with a single movement of the piston 212.

According to a preferred embodiment, the pulmonary ventilator 1 comprises a first inlet 10, configured for being placed in fluid communication with a source of a first gas under pressure. The source of the first gas under pressure is, in the present case, a primary source external to the pulmonary ventilator 1, for example forming part of a distribution system of the hospital/healthcare facility wherein the pulmonary ventilator 1 can be installed and the inlet 31 of the actuation chamber 3 is configured for being selectively put in fluid communication with the first inlet 10, to selectively receive the abovesaid first gas under pressure, through said device 22 for adjusting the entering flow.

The pulmonary ventilator 1 also has a second inlet 13, configured for receiving from the outside a second gas under pressure, for example delivered by a primary source external to the pulmonary ventilator 1, for example forming part of a distribution system of the hospital/healthcare facility wherein the pulmonary ventilator 1 can be installed.

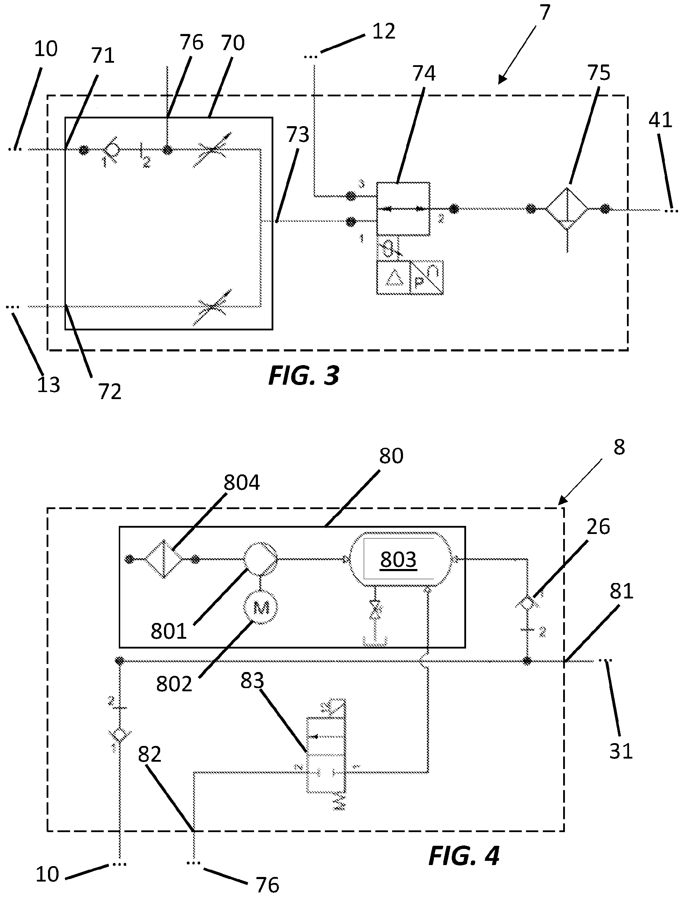

The pulmonary ventilator 1 comprises a mixing circuit 7 (Figure 3) including a mixing chamber 70, a first inlet 71 in said mixing chamber 70, a second inlet 72 in the mixing chamber 70 and one outlet 73 from said mixing chamber. The first inlet 71 of the mixing chamber 70 is in fluid communication with the first inlet 10 of the pulmonary ventilator 1 and is configured to receive the first gas under pressure, when delivered by the primary source. The second inlet 72 of the mixing chamber 70 is in fluid communication with the second inlet 13 of the pulmonary ventilator 1 and is configured to receive the second gas under pressure, when delivered by a respective primary source. The outlet 73 of the mixing chamber is in fluid communication with the inlet 41 of the ventilation chamber 4 of the cylinder-piston unit 21 of the adjustment circuit 2.

The mixing circuit 7 is configured for mixing the first gas under pressure and the second gas under pressure therebetween, in the mixing chamber 70, thereby obtaining the ventilation gas. Advantageously, the first gas under pressure comprises medical air or a mixture of helium and oxygen (for example heliox) and the second gas under pressure comprises oxygen.

Downstream of the outlet 73 of the mixing chamber, the mixing circuit 7 also comprises one device 74 for adjusting pressure, for example one valve for detecting and adjusting the pressure of the ventilation gas directed to the ventilation chamber 4, controlled by the acquisition and processing unit 6 and in fluid communication with the exhaust outlet 12 of the pulmonary ventilator 1, and optionally a dehumidifier filter 75, so that the ventilation gas sent to the ventilation chamber 4 has the correct pressure and humidity level.

According to another particularly advantageous aspect, the pulmonary ventilator 1 also comprises one auxiliary circuit 8 for supplying the first gas under pressure (Figure 4). Said auxiliary circuit 8 comprises one pressurized storage group 80 for the first gas and a first outlet 81. The first outlet 81 of the auxiliary circuit 8 is in fluid communication with the first gas under pressure stored in the storage group 80 on one side and, on the other side, is selectively in fluid communication with the inlet 31 of the actuation chamber 3, through the device 22 for adjusting the flow entering the actuation chamber 3 controlled by the acquisition and processing unit 6.

The pressurized storage group 80 for the first gas comprises, according to a preferred embodiment, a membrane pump 801 controlled by a motor unit 802 and configured to draw in air from the outside. The storage unit 80 also comprises one tank 803 downstream of the membrane pump, configured to store the air drawn in by the same, and optionally also one filter 804, connected upstream of the membrane pump 801, and configured to filter the air coming from the outside before it is aspirated by the membrane pump. The first outlet 81 of the auxiliary circuit 8 is, therefore, in fluid communication with the tank 803, optionally through a one-way valve 26. The auxiliary circuit 8 is configured for supplying the first gas under pressure (i.e. air, optionally filtered, and under pressure) when the first gas under pressure (i.e., medical air) does not enter the pulmonary ventilator 1 at the first inlet 10 thereof, for example because the primary source of the first gas under pressure is disconnected from the first inlet 10 thereof or due to some malfunction.

According to another advantageous aspect, the mixing circuit 7 also comprises a third inlet 76 in the mixing chamber 70 and the auxiliary circuit 8 for supplying the first pressurized gas comprises at least a second outlet 82.The second outlet 82 of the auxiliary circuit 8 is, on the one hand, in fluid communication with the tank 803 of the storage group 80 and, on the other hand, selectively in fluid communication with the third inlet 76 of the mixing circuit 7, optionally by means of a device 83 for adjusting the flow, optionally a two-way valve, included in the auxiliary circuit 8 between the tank 803 and the second outlet 82. The opening-closing of the second outlet 82 is operatively controlled by the acquisition and processing unit 6 so that the second outlet 82 of the auxiliary circuit 8 is closed when the first pressurized gas enters the pulmonary ventilator 1 at its first inlet 10, and is instead open (by switching the two-way valve 83 by the acquisition and processing unit 6, if provided ), when the first gas under pressure does not enter the pulmonary ventilator 1 at its first inlet 10, for example because the primary source of the first gas under pressure is disconnected from its first inlet 10 or exhausted. In this case, the first gas under pressure contained in the tank 803 will be able to flow into the mixing chamber 70 of the mixing circuit, as well as into the operating chamber 31 if the flow entering the adjusting device 22 is in the correct position (i.e., open).

With this configuration of the auxiliary circuit 8, it is completely clear that the pulmonary ventilator 1 can be easily used as a portable pulmonary ventilator, for example by disconnecting the first inlet 10 and the second inlet 13 of the pulmonary ventilator 1 from the distribution network of the hospital/healthcare facility in which it is located, activating the membrane pump 801 and connecting the second inlet 13 of the pulmonary ventilator to an auxiliary source of medical oxygen, for example to an oxygen cylinder. In this case, the person skilled in the art will have no difficulty in understanding how each component of the pulmonary ventilator 1, which requires an electrical power supply for its operation, such as for example the acquisition and processing unit 6, will be configured to be able to be powered not only by a connection of the pulmonary ventilator 1 to the electrical distribution network of the hospital/healthcare facility in which the ventilator is located, but also by an optionally rechargeable battery which is incorporated in the pulmonary ventilator 1. In this regard, it should be noted that according to a preferred embodiment, the pulmonary ventilator 1 comprises a pair of rechargeable batteries, wherein each battery is electrically connected to each component of the pulmonary ventilator 1 which requires an electrical power supply for its operation , and can be activated as an alternative, to prevent the malfunction of the battery from compromising the operation of the pulmonary ventilator when the ventilator 1 is not powered by the electrical distribution network of the structure.

According to another advantageous aspect, the pulmonary ventilator 1 also comprises a bypass circuit 200 for the ventilation gas, connected between the inlet and the outlet of the ventilation chamber 4 of the cylinder-piston unit 2, in particular upstream of the adjustment device 24 and downstream of the adjustment device 25, as well as connected, at the adjustment device 25 also to the exhaust outlet 12 of the pulmonary ventilator 1. Such bypass circuit 200 is operatively connected to the acquisition and processing unit 6 and configured to provide and alternative passage for the ventilation gas with respect to the adjustment circuit 2, to allow ventilation of a patient through the so-called high-flow technique, wherein the ventilation gas flow rate is over a certain threshold, optionally equal to or greater than 80 I/ minute. According to a preferred embodiment, the bypass circuit 200 comprises at least one proportional flow control valve.

The pulmonary ventilator 1 described above can be used to supply a desired volume Vd of ventilation gas at a desired pressure Pd according to a non-claimed method. Such a non-claimed method comprises the following operational steps:
A. arranging a pulmonary ventilator as described above;
B. putting in fluid communication the first inlet 31 of the actuation chamber 3 with the first gas under pressure and the inlet 41 of the ventilation chamber 4 with the ventilation gas under pressure;
C. supplying one quantity of ventilation gas in the ventilation chamber 4, so that the volume occupied by the ventilation gas in the ventilation chamber 4 is at least corresponding or greater than said desired volume Vd; and then
D. delivering at least that desired volume Vd of ventilation gas in said ventilation chamber 4 toward said ventilation outlet 11 of said pulmonary ventilator 1 at a preset desired pressure Pd.

According to a preferred embodiment, steps C and D of the above-described non-claimed method are repeated sequentially (step C before step D) and cyclically, for example for each respiratory act of a patient connected to the ventilator. More particularly, each delivery at step D of the desired volume Vd of the ventilation gas corresponds, in terms of time, to a respective inspiration of the patient.

Going back to the steps of the non-claimed method, it will be noted that step B comprises, in turn:
- putting in fluid communication the first inlet 10 of the pulmonary ventilator 1 with the primary source of the first gas under pressure and opening the device 22 for adjusting the flow entering the actuation chamber 3 keeping closed the device 23 for adjusting the flow leaving the actuation chamber 3, and putting into fluid communication the second inlet 13 of the pulmonary ventilator 1 with the primary source of the second gas under pressure and opening the device 24 for adjusting the flow entering the ventilation chamber 4 while keeping closed the device 25 for adjusting the floe leaving the ventilation chamber 4, or
- putting into fluid communication the storage group (in particular the tank 803) of the supplying auxiliary circuit 8, containing the first gas under pressure both with the actuation chamber 3, by opening the device 22 for adjusting the flow entering the actuation chamber 3 and keeping closed the device 23 for adjusting the flow leaving the actuation chamber 3, and with the mixing circuit 7 through the third inlet 76 thereof, by opening the device 83 for adjusting the flow upstream of the second outlet 82, and putting into fluid communication the second inlet 13 of the pulmonary ventilator 13 with an auxiliary source of the second gas under pressure, for example one tank, and opening the device 24 for adjusting the flow entering the ventilation chamber 4 while keeping closed the device 25 for adjusting the flow leaving the ventilation chamber 4.

With reference to step C of the non-claimed method, this comprises, through the acquisition and processing unit 6:
C.1 acquiring and processing input signals regarding the position of the piston 212 in the cylinder 211, optionally continuously or at steps, through the position detector 29; and
C.2 if necessary, providing output signals for adjusting the pressure of the first gas in the actuation chamber 3 and the pressure of the ventilation gas in the ventilation chamber 4 through controlled opening-closing of the respective devices for adjusting the incoming and outgoing flow, thereby obtaining the movement of the piston 212 in the cylinder 211, until the volume of the ventilation chamber 4 is at least equal to the desired volume Vd.

As will be noted, the volume in the ventilation chamber 4 can be controlled very precisely as the geometry of the cylinder-piston unit 21 is known a priori and the position of the piston 212 can be detected by the acquisition and processing unit 6 through the position detector 29. In this way, once the desired volume Vd is known, it is easy to determine, thanks to the acquisition and processing unit 6 how much the piston 212 must be moved in the cylinder and, therefore, how much the pressures between the actuation chamber 3 and the ventilation chamber 4 must be changed, to obtain this effect.

With reference to step D of the non-claimed method, step D comprises, through the acquisition and processing unit 6:
D.1 acquiring and processing input signals regarding the position of piston 212 in the cylinder 211, optionally continuously or at steps, through the position detector 29; and
D.2 if necessary, providing output signals for adjusting the pressure of the first gas in the actuation chamber 3 and the pressure of the ventilation gas in the ventilation chamber 4 through controlled opening-closing of the respective devices for adjusting the incoming and outgoing flow, thereby obtaining the movement of the piston 212 in the cylinder 211, until the volume of the ventilation chamber 4 is reduced of a quantity equal to the desired volume Vd.

Clearly the same considerations made above with reference to the precision with which it is possible to control the volume of the measurement chamber apply, given the presence of the magnetized piston 212 and the position detector 28.

The person skilled in the art will have no difficulty in understanding how steps C.2 and D.2 require the control, through the acquisition and processing unit 6, of the devices (22, 23, 24 and 25) for adjusting the flow entering and leaving each chamber of the unit of the cylinder-piston unit 21, so that the first gas and the ventilation gas reach the pressures necessary to determine, due to the their difference, the displacement of the cylinder 212 in the desired direction.

The non-claimed method also comprises at step E, through the acquisition and processing unit 6:
E.3 sending output signals for adjusting the pressure of the first gas in the actuation chamber 3 and the pressure of the ventilation gas in the ventilation chamber 4, so that during step D the ventilation gas leaves the ventilation chamber 4 at the desired pressure Pd.

This pressure control comprises adjusting, through the acquisition and processing unit 6, the opening-closing of the device 25 for adjusting the flow leaving the ventilation chamber 4.

With this configuration of the pulmonary ventilator 1 and with the non-claimed method described above is also possible to use the pulmonary ventilator 1 in cases where a patient demand for ventilation gas of is higher than the maximum physical volume that the ventilation chamber 4 can have. In fact, by suitably adjusting the devices (22, 23, 24 and 25) for adjusting the flow entering and leaving each chamber of the cylinder-piston unit 21, it is possible to compress the ventilation gas in the ventilation chamber 4 to store a greater quantity thereof, corresponding to the desired volume Vd required by the patient.

It is evident that the pulmonary ventilator described above, and its operating non-claimed method solve the drawbacks indicated in the introduction. The particular configuration of the pulmonary ventilator 1, in fact, with the magnetized piston 212 completely closed inside the cylinder 211, the position detector 29 external to the cylinder 211 and the devices for (22, 23, 24, 25) adjusting the flow entering and leaving the actuation chamber and the ventilation chamber, allows controlling easily and in a more precise and reliable way than the traditional ventilators, both the pressure and the volume of the gas delivered to a patient. The particular configuration of the piston 212 also allows avoiding contamination from the outside of the gases circulating in the pulmonary ventilator. The absence of an external mechanical actuation system for the piston 212, in the cylinder-piston 21 unit, additionally reduces the overall dimensions of the pulmonary ventilator 1 as well as its consumption and the presence of the auxiliary circuit for the supply of the first gas makes it easily transportable, therefore portable. Not only that, being possible to easily control the pressures in the actuation chamber and ventilation chamber, it is possible to use the pulmonary ventilator even when the tidal volume required by a patient is higher than the maximum physical volume that the ventilation chamber 4 of the cylinder-piston unit21 can reach.

In the foregoing the preferred embodiments have been described and variants of the present invention have been suggested, but it is to be understood that those skilled in the art will be able to make modifications and changes without thereby departing from the relative scope of protection, as defined by the attached claims.

For example, in the preferred embodiment described above, the one-way valves 26 could be replaced by elements that are equivalent from a technical point of view, which allow maintaining the unidirectionality of the flow of the actuation gas and ventilation gas, as described above.

Moreover, for example, in the mixing chamber 70, downstream of the respective inlets 71 and 72, respective devices for adjusting the flow of the first and second gas can be provided, operatively connected to the acquisition and processing unit 6 and operable by the same both manually and automatically, to control the mixing of these gases in the ventilation gas.

Furthermore, the pulmonary ventilator 1 of the present invention can be configured to allow introducing further substances, for example nebulized drugs or sedatives to be administered by respiratory route to the patient, into the mixture of the first and second gas.

## Claims

1. Pulmonary ventilator (1) configured for delivering to a patient, for each respiratory act, a desired volume (Vd) of a ventilation gas at a desired pressure (Pd), said pulmonary ventilator (1) having a ventilation outlet (11), configured for being put in fluid communication with respiratory tract of said patient, and an exhaust outlet (12), in communication with the outside of said pulmonary ventilator (1), said pulmonary ventilator (1) comprising at least one adjustment circuit (2), having:
- a cylinder-piston unit (21) having a cylinder (211) and a piston (212) which delimit:
- an actuation chamber (3), having an inlet (31) and an outlet (32), wherein said inlet (31) of said actuation chamber (3) is configured for selectively receiving a first gas under pressure, and said outlet (32) of said actuation chamber (3) is in fluid communication with said exhaust outlet (12); and
- a ventilation chamber (4), having an inlet (41) and an outlet (42), wherein said inlet (41) of said ventilation chamber (4) is configured for selectively receiving said ventilation gas under pressure to be provided to said patient, and said outlet (42) of said ventilation chamber (4) is in fluid communication with said ventilation outlet (11) of said pulmonary ventilator (1);
- at least one device (22) for adjusting the flow entering into said actuation chamber (3) and at least one a device (23) for adjusting the flow leaving said actuation chamber (3), at least one device (24) for adjusting the flow entering into said ventilation chamber (4) and at least one device (25) for adjusting the flow leaving said ventilation chamber (4);
- at least one pressure detecting device (27), configured for detecting in use the pressure of said first gas in said actuation chamber (3) and another pressure detecting device (28), configured for detecting in use the pressure value of said ventilation gas in said ventilation chamber (4);
- one position detector (29), configured for detecting the position of said piston (212) inside said cylinder (211);
- one acquisition and processing unit (6), operatively connected to said position detector (29), each device (22, 23, 24, 25) for adjusting the flow and each pressure detecting device (27, 28)
wherein said acquisition and processing unit (6) is configured for:
- acquiring and processing input signals regarding the position of said piston (212) in said cylinder (212), the pressure of said first gas in said actuation chamber (3) and the pressure of said ventilation gas in said ventilation chamber (4); and
- providing output signals for controlling the opening-closing of each device (22, 23) for adjusting the flow entering into and leaving said actuation chamber (3) and the opening-closing of each device (24, 25) for adjusting the flow entering into and leaving said ventilation chamber (4), based on said position of said piston (212) in said cylinder (211), said pressure of said first gas in said actuation chamber (3) and said pressure of said ventilation gas in said ventilation chamber (4), so that one quantity of said ventilation gas corresponding to at least said desired volume (Vd) enters into said ventilation chamber (4) and then said desired volume (Vd) of said ventilation gas leaves said ventilation chamber (4) toward said ventilation outlet (11) at said desired pressure (Pd).

2. Pulmonary ventilator (1) according to claim 1, wherein said piston (212) is a magnetized piston, configured for being moved inside said cylinder (211) due to the pressure difference between said first gas in said actuation chamber (3) and said ventilation gas in said ventilation chamber (4).

3. Pulmonary ventilator (1) according to claim 2, wherein said position detector (29) is externally fixed to said cylinder (211) and is configured for magnetically detecting the position of said piston (212) in said cylinder (211).

4. Pulmonary ventilator (1) according to any previous claim, comprising at least one first inlet (10), configured for being placed in fluid communication with a source of a first gas under pressure, wherein said source is a primary source external to said pulmonary ventilator (1) and wherein said inlet (31) of said actuation chamber (3) is configured for being selectively placed in fluid communication with said first inlet (10) of said pulmonary ventilator (1) for selectively receiving said first gas under pressure.

5. Pulmonary ventilator (1) according to claim 4, having one second inlet (13) and comprising at least one mixing circuit (7), said at least one mixing circuit (7) including:
- one mixing chamber (70);
- a first inlet (71) in said mixing chamber (70), said first inlet (71) of said mixing chamber being in fluid communication with said first inlet (10) of said pulmonary ventilator (1) and configured for receiving said first gas under pressure, when provided by said primary source;
- a second inlet (72) in said mixing chamber (70), said second inlet (72) of said mixing chamber (70) being in fluid communication with said second inlet (13) of said pulmonary ventilator (1) and configured for receiving a second gas under pressure; and
- a first outlet (73) of said mixing chamber (70), in fluid communication with said inlet (41) of said ventilation chamber (4),
said at least one mixing circuit (7) being operatively controlled by said acquisition and processing unit (6) and configured for mixing said first gas under pressure and sad second gas under pressure therebetween, thereby obtaining said ventilation gas.

6. Pulmonary ventilator (1) according to any previous claim, comprising one auxiliary circuit (8) for supplying said first gas under pressure, said auxiliary circuit (8) comprising:
- one pressurized storage group (80) of said first gas under pressure; and
- a first outlet (81), in fluid communication with said storage group (80) on one side and, on the other side, selectively in fluid communication with said inlet (31) of said actuation chamber (3), said first outlet (81) of said auxiliary circuit (8) being operatively controlled by said acquisition and processing unit (6) and configured for being closed, when said primary source of said first gas under pressure is connected to said first inlet (10), or opened, when said primary source of said first gas under pressure is disconnected from said first inlet (10).

7. Pulmonary ventilator (1) according to claim 5 and 6, wherein said mixing circuit (7) comprises a third inlet (76) in said mixing chamber (70), and wherein said auxiliary circuit (8) for supplying said first gas under pressure comprises at least one second outlet (82), in fluid communication with said storage group (80) on one side and, on the other side, in fluid communication with said third inlet (76) of said mixing circuit (7), said second outlet (82) being operatively controlled by said acquisition and processing unit (6) and configured for being closed, when said primary source of said first gas under pressure is connected to said first inlet (10), or opened, when said primary source of said first gas under pressure is disconnected from said first inlet (10), so that said first gas under pressure in said storage group (80) can be supplied in said mixing chamber (70).

8. Pulmonary ventilator (1) according to any previous claim, wherein said acquisition and processing unit (6) comprises at least two programmable electronic systems in communication with each other, which are configured to implement a hardware and control redundancy.

## Patentansprüche

1. Lungenbeatmungsgerät (1), das dazu konfiguriert ist, einem Patienten für jeden Atemvorgang ein gewünschtes Volumen (Vd) eines Beatmungsgases mit einem gewünschten Druck (Pd) zuzuführen, wobei das Lungenbeatmungsgerät (1) einen Beatmungsauslass (11) und einen Abgasauslass (12) aufweist, wobei der Beatmungsauslass (11) dazu konfiguriert ist, mit dem Atemweg des Patienten in Fluidverbindung gebracht zu werden, wobei der Abgasauslass (12) mit der Außenseite des Lungenbeatmungsgeräts (1) in Verbindung steht, wobei das Lungenbeatmungsgerät (1) mindestens einen Einstellkreis (2) umfasst, der Folgendes aufweist:
- eine Zylinder-Kolben-Einheit (21) mit einem Zylinder (211) und einem Kolben (212), die Folgendes begrenzen:
- eine Betätigungskammer (3) mit einem Einlass (31) und einem Auslass (32), wobei der Einlass (31) der Betätigungskammer (3) dazu konfiguriert ist, selektiv ein erstes unter Druck stehendes Gas aufzunehmen, und der Auslass (32) der Betätigungskammer (3) mit dem Abgasauslass (12) in Fluidverbindung steht; und
- eine Beatmungskammer (4) mit einem Einlass (41) und einem Auslass (42), wobei der Einlass (41) der Beatmungskammer (4) dazu konfiguriert ist, selektiv das Beatmungsgas unter Druck aufzunehmen, das dem Patienten bereitgestellt werden soll, und der Auslass (42) der Beatmungskammer (4) mit dem Beatmungsauslass (11) des Lungenbeatmungsgeräts (1) in Fluidverbindung steht;
- mindestens eine Vorrichtung (22) zum Einstellen der Strömung, die in die Betätigungskammer (3) eintritt, und mindestens eine Vorrichtung (23) zum Einstellen der Strömung, die die Betätigungskammer (3) verlässt, mindestens eine Vorrichtung (24) zum Einstellen der Strömung, die in die Beatmungskammer (4) eintritt, und mindestens eine Vorrichtung (25) zum Einstellen der Strömung, die die Beatmungskammer (4) verlässt;
- mindestens eine Druckerfassungsvorrichtung (27), die dazu konfiguriert ist, im Gebrauch den Druck des ersten Gases in der Betätigungskammer (3) zu erfassen, und eine weitere Druckerfassungsvorrichtung (28), die dazu konfiguriert ist, im Gebrauch den Druckwert des Beatmungsgases in der Beatmungskammer (4) zu erfassen;
- einen Positionsdetektor (29), der dazu konfiguriert ist, die Position des Kolbens (212) innerhalb des Zylinders (211) zu erfassen;
- eine Erfassungs- und Verarbeitungseinheit (6), die mit dem Positionsdetektor (29), jeder Vorrichtung (22, 23, 24, 25) zum Einstellen der Strömung und jeder Druckerfassungsvorrichtung (27, 28) wirkverbunden ist,
wobei die Erfassungs- und Verarbeitungseinheit (6) für Folgendes konfiguriert ist:
- Erfassen und Verarbeiten von Eingangssignalen bezüglich der Position des Kolbens (212) in dem Zylinder (212), des Drucks des ersten Gases in der Betätigungskammer (3) und des Drucks des Beatmungsgases in der Beatmungskammer (4); und
- Bereitstellen von Ausgangssignalen zum Steuern des Öffnens/Schließens jeder Vorrichtung (22, 23) zum Einstellen der Strömung, die in die Betätigungskammer (3) eintritt und diese verlässt, und des Öffnens/Schließens jeder Vorrichtung (24, 25) zum Einstellen der Strömung, die in die Beatmungskammer (4) eintritt und diese verlässt, basierend auf der Position des Kolbens (212) in dem Zylinder (211), dem Druck des ersten Gases in der Betätigungskammer (3) und dem Druck des Beatmungsgases in der Beatmungskammer (4), so dass eine Menge des Beatmungsgases, die mindestens dem gewünschten Volumen (Vd) entspricht, in die Beatmungskammer (4) eintritt und dann das gewünschte Volumen (Vd) des Beatmungsgases die Beatmungskammer (4) 10 zu dem Beatmungsauslass (11) mit dem gewünschten Druck (Pd) hin verlässt.

2. Lungenbeatmungsgerät (1) nach Anspruch 1, wobei der Kolben (212) ein magnetisierter Kolben ist, der dazu konfiguriert ist, aufgrund der Druckdifferenz zwischen dem ersten Gas in der Betätigungskammer (3) und dem Beatmungsgas in der Beatmungskammer (4) innerhalb des Zylinders (211) bewegt zu werden.

3. Lungenbeatmungsgerät (1) nach Anspruch 2, wobei der Positionsdetektor (29) außen an dem Zylinder (211) befestigt ist und dazu konfiguriert ist, die Position des Kolbens (212) in dem Zylinder (211) magnetisch zu erfassen.

4. Lungenbeatmungsgerät (1) nach einem der vorhergehenden Ansprüche, umfassend mindestens einen ersten Einlass (10), der dazu konfiguriert ist, mit einer Quelle eines ersten Gases unter Druck in Fluidverbindung gebracht zu werden,
wobei die Quelle eine primäre Quelle außerhalb des Lungenbeatmungsgeräts (1) ist und wobei der Einlass (31) der Betätigungskammer (3) dazu konfiguriert ist, selektiv mit dem ersten Einlass (10) des Lungenbeatmungsgeräts (1) in Fluidverbindung gebracht zu werden, um selektiv das erste Gas unter Druck aufzunehmen.

5. Lungenbeatmungsgerät (1) nach Anspruch 4, das einen zweiten Einlass (13) aufweist und
mindestens einen Mischkreis (7) umfasst, wobei der mindestens eine Mischkreis (7) Folgendes umfasst:
- eine Mischkammer (70);
- einen ersten Einlass (71) in der Mischkammer (70), wobei der erste Einlass (71) der Mischkammer mit dem ersten Einlass (10) des Lungenbeatmungsgeräts (1) in Fluidverbindung steht und dazu konfiguriert ist, das erste Gas unter Druck aufzunehmen, wenn es von der primären Quelle bereitgestellt wird;
- einen zweiten Einlass (72) in der Mischkammer (70), wobei der zweite Einlass (72) der Mischkammer (70) mit dem zweiten Einlass (13) des Lungenbeatmungsgeräts (1) in Fluidverbindung steht und dazu konfiguriert ist, ein zweites Gas unter Druck aufzunehmen; und
- einen ersten Auslass (73) der Mischkammer (70), der mit dem Einlass (41) der Beatmungskammer (4) in Fluidverbindung steht, wobei der mindestens eine Mischkreis (7) von der Erfassungs- und Verarbeitungseinheit (6) wirkgesteuert wird und dazu konfiguriert ist, das erste Gas unter Druck und das zweite Gas unter Druck dazwischen zu mischen, wodurch das Beatmungsgas erhalten wird.

6. Lungenbeatmungsgerät (1) nach einem der vorhergehenden Ansprüche, umfassend einen Hilfskreis (8) zum Zuführen des ersten Gases unter Druck, wobei der Hilfskreis (8) Folgendes umfasst:
- eine unter Druck stehende Speichergruppe (80) des ersten Gases unter Druck; und
- einen ersten Auslass (81), der auf einer Seite mit der Speichergruppe (80) in Fluidverbindung steht und auf der anderen Seite selektiv mit dem Einlass (31) der Betätigungskammer (3) in Fluidverbindung steht, wobei der erste Auslass (81) des Hilfskreises (8) von der Erfassungs- und Verarbeitungseinheit (6) wirkgesteuert wird und dazu konfiguriert ist, geschlossen zu werden, wenn die primäre Quelle des ersten Gases unter Druck mit dem ersten Einlass (10) verbunden ist, oder geöffnet zu werden, wenn die primäre Quelle des ersten Gases unter Druck von dem ersten Einlass (10) getrennt ist.

7. Lungenbeatmungsgerät (1) nach Anspruch 5 und 6, wobei der Mischkreis (7) einen dritten Einlass (76) in der Mischkammer (70) umfasst und wobei der Hilfskreis (8) zum Zuführen des ersten Gases unter Druck mindestens einen zweiten Auslass (82) umfasst, der auf einer Seite mit der Speichergruppe (80) in Fluidverbindung steht und auf der anderen Seite mit dem dritten Einlass (76) des Mischkreises (7) in Fluidverbindung steht, wobei der zweite Auslass (82) von der Erfassungs- und Verarbeitungseinheit (6) wirkgesteuert wird und dazu konfiguriert ist, geschlossen zu werden, wenn die primäre Quelle des ersten Gases unter Druck mit dem ersten Einlass (10) verbunden ist, oder geöffnet zu werden, wenn die primäre Quelle des ersten Gases unter Druck von dem ersten Einlass (10) getrennt ist, so dass das erste Gas unter Druck in der Speichergruppe (80) in die Mischkammer (70) zugeführt werden kann.

8. Lungenbeatmungsgerät (1) nach einem der vorhergehenden Ansprüche, wobei die Erfassungs- und Verarbeitungseinheit (6) mindestens zwei programmierbare elektronische Systeme in Kommunikation miteinander umfasst, die dazu konfiguriert sind, eine Hardware- und Steuerredundanz zu implementieren.

## Revendications

1. Ventilateur pulmonaire (1) configuré pour administrer à un patient, pour chaque acte respiratoire, un volume souhaité (Vd) d'un gaz de ventilation à une pression souhaitée (Pd), ledit ventilateur pulmonaire (1) ayant une sortie de ventilation (11), configurée pour être mise en communication fluidique avec les voies respiratoires dudit patient, et une sortie d'évacuation (12), en communication avec l'extérieur dudit ventilateur pulmonaire (1), ledit ventilateur pulmonaire (1) comprenant au moins un circuit de réglage (2), ayant:
- une unité cylindre-piston (21) ayant un cylindre (211) et un piston (212) qui délimitent:
- une chambre d'actionnement (3), ayant une entrée (31) et une sortie (32), où ladite entrée (31) de ladite chambre d'actionnement (3) est configurée pour recevoir de manière sélective un premier gaz sous pression, et ladite sortie (32) de ladite chambre d'actionnement (3) est en communication fluidique avec ladite sortie d'évacuation (12); et
- une chambre de ventilation (4), ayant une entrée (41) et une sortie (42), où ladite entrée (41) de ladite chambre de ventilation (4) est configurée pour recevoir de manière sélective ledit gaz de ventilation sous pression à fournir audit patient, et ladite sortie (42) de ladite chambre de ventilation (4) est en communication fluidique avec ladite sortie de ventilation (11) dudit ventilateur pulmonaire (1);
- au moins un dispositif (22) pour régler le débit entrant dans ladite chambre d'actionnement (3) et au moins un dispositif (23) pour régler le débit sortant de ladite chambre d'actionnement (3), au moins un dispositif (24) pour régler le débit entrant dans ladite chambre de ventilation (4) et au moins un dispositif (25) pour régler le débit sortant de ladite chambre de ventilation (4);
- au moins un dispositif de détection de pression (27), configuré pour détecter en cours d'utilisation la pression dudit premier gaz dans ladite chambre d'actionnement (3) et un autre dispositif de détection de pression (28), configuré pour détecter en cours d'utilisation la valeur de pression dudit gaz de ventilation dans ladite chambre de ventilation (4);
- un détecteur de position (29), configuré pour détecter la position dudit piston (212) à l'intérieur dudit cylindre (211);
- une unité d'acquisition et de traitement (6), reliée de manière fonctionnelle audit détecteur de position (29), à chaque dispositif (22, 23, 24, 25) pour régler le débit et à chaque dispositif de détection de pression (27, 28), où ladite unité d'acquisition et de traitement (6) est configurée pour:
- acquérir et traiter des signaux d'entrée concernant la position dudit piston (212) dans ledit cylindre (212), la pression dudit premier gaz dans ladite chambre d'actionnement (3) et la pression dudit gaz de ventilation dans ladite chambre de ventilation (4); et
- fournir des signaux de sortie pour commander l'ouverture-fermeture de chaque dispositif (22, 23) pour régler le débit entrant dans et sortant de ladite chambre d'actionnement (3) et l'ouverture-fermeture de chaque dispositif (24, 25) pour régler le débit entrant dans et sortant de ladite chambre de ventilation (4), sur la base de ladite position dudit piston (212) dans ledit cylindre (211), de ladite pression dudit premier gaz dans ladite chambre d'actionnement (3) et de ladite pression dudit gaz de ventilation dans ladite chambre de ventilation (4), de sorte qu'une quantité dudit gaz de ventilation correspondant à au moins ledit volume souhaité (Vd) entre dans ladite chambre de ventilation (4) et ensuite ledit volume souhaité (Vd) dudit gaz de ventilation sort de ladite chambre de ventilation (4) vers ladite sortie de ventilation (11) à ladite pression souhaitée (Pd).

2. Ventilateur pulmonaire (1) selon la revendication 1, dans lequel ledit piston (212) est un piston magnétisé, configuré pour être déplacé à l'intérieur dudit cylindre (211) en raison de la différence de pression entre ledit premier gaz dans ladite chambre d'actionnement (3) et ledit gaz de ventilation dans ladite chambre de ventilation (4).

3. Ventilateur pulmonaire (1) selon la revendication 2, dans lequel ledit détecteur de position (29) est extérieurement fixé audit cylindre (211) et est configuré pour détecter magnétiquement la position dudit piston (212) dans ledit cylindre (211).

4. Ventilateur pulmonaire (1) selon l'une des revendications précédentes, comprenant au moins une première entrée (10), configurée pour être placée en communication fluidique avec une source d'un premier gaz sous pression, où ladite source est une source primaire externe audit ventilateur pulmonaire (1) et où ladite entrée (31) de ladite chambre d'actionnement (3) est configurée pour être placée de manière sélective en communication fluidique avec ladite première entrée (10) dudit ventilateur pulmonaire (1) afin de recevoir de manière sélective ledit premier gaz sous pression.

5. Ventilateur pulmonaire (1) selon la revendication 4, ayant une deuxième entrée (13) et comprenant au moins un circuit de mélange (7), ledit au moins un circuit de mélange (7) incluant:
- une chambre de mélange (70);
- une première entrée (71) dans ladite chambre de mélange (70), ladite première entrée (71) de ladite chambre de mélange étant en communication fluidique avec ladite première entrée (10) dudit ventilateur pulmonaire (1) et configurée pour recevoir ledit premier gaz sous pression, lorsqu'il est fourni par ladite source primaire;
- une deuxième entrée (72) dans ladite chambre de mélange (70), ladite deuxième entrée (72) de ladite chambre de mélange (70) étant en communication fluidique avec ladite deuxième entrée (13) dudit ventilateur pulmonaire (1) et configurée pour recevoir un second gaz sous pression; et
- une première sortie (73) de ladite chambre de mélange (70), en communication fluidique avec ladite entrée (41) de ladite chambre de ventilation (4),
ledit au moins un circuit de mélange (7) étant commandé de manière fonctionnelle par ladite unité d'acquisition et de traitement (6) et configuré pour mélanger ledit premier gaz sous pression et ledit second gaz sous pression entre eux, obtenant ainsi ledit gaz de ventilation.

6. Ventilateur pulmonaire (1) selon l'une des revendications précédentes, comprenant un circuit auxiliaire (8) pour fournir ledit premier gaz sous pression, ledit circuit auxiliaire (8) comprenant:
- un groupe de stockage sous pression (80) dudit premier gaz sous pression; et
- une première sortie (81), en communication fluidique avec ledit groupe de stockage (80) sur un côté et, sur l'autre côté, sélectivement en communication fluidique avec ladite entrée (31) de ladite chambre d'actionnement (3), ladite première sortie (81) dudit circuit auxiliaire (8) étant commandée de manière fonctionnelle par ladite unité d'acquisition et de traitement (6) et configurée pour être fermée, lorsque ladite source primaire dudit premier gaz sous pression est connectée à ladite première entrée (10), ou ouverte, lorsque ladite source primaire dudit premier gaz sous pression est déconnectée de ladite première entrée (10).

7. Ventilateur pulmonaire (1) selon les revendications 5 et 6, dans lequel ledit circuit de mélange (7) comprend une troisième entrée (76) dans ladite chambre de mélange (70), et dans lequel ledit circuit auxiliaire (8) de fourniture dudit premier gaz sous pression comprend au moins une seconde sortie (82), en communication fluidique avec ledit groupe de stockage (80) sur un côté et, sur l'autre côté, en communication fluidique avec ladite troisième entrée (76) dudit circuit de mélange (7), ladite seconde sortie (82) étant commandée de manière fonctionnelle par ladite unité d'acquisition et de traitement (6) et configurée pour être fermée, lorsque ladite source primaire dudit premier gaz sous pression est connectée à ladite première entrée (10), ou ouverte, lorsque ladite source primaire dudit premier gaz sous pression est déconnectée de ladite première entrée (10), de sorte que ledit premier gaz sous pression dans ledit groupe de stockage (80) puisse être fourni dans ladite chambre de mélange (70).

8. Ventilateur pulmonaire (1) selon l'une des revendications précédentes, dans lequel ladite unité d'acquisition et de traitement (6) comprend au moins deux systèmes électroniques programmables en communication l'un avec l'autre, qui sont configurés pour mettre en œuvre une redondance matérielle et de commande.
